# EUROPEAN PATENT SPECIFICATION

(11) **EP 3 083 992 B1**
(45) Date of publication and mention of the grant of the patent: **17.01.2018**
(21) Application number: 14821146.9
(22) Date of filing: 18.12.2014
(51) Int. Cl.: C12Q 1/68

(54) **A METHOD FOR CODING OF MULTIPLE PCR REACTIONS FOR ASSAY RECOGNITION**
VERFAHREN ZUR CODIERUNG VON MEHREREN PCR-REAKTIONEN ZUR TESTERKENNUNG
PROCÉDÉ POUR CODER PLUSIEURS RÉACTIONS PCR AFIN DE POUVOIR RECONNAÎTRE LES ANALYSES

(30) Priority: 20.12.2013 US 201361918892 P
(43) Date of publication of application: 26.10.2016
(73) Proprietor: Roche Diagnostics GmbH, 68305 Mannheim (DE); F. Hoffmann-La Roche AG, 4070 Basel (CH)
(72) Inventor: GUPTA, Amar, Danville, California 94526 (US); JANSSEN, Kevin, Philadelphia, PA 19104 (US); NEWTON, Nicolas, Oakland, California 94619 (US); SCHOENBRUNNER, Nancy, Charlestown, Massachusetts 02129 (US)
(74) Representative: Schwarz, Ralf
(86) International application number: PCT/EP2014/078406
(87) International publication number: WO 2015/091756

(56) References cited:
- EP-A1- 2 130 929
- WO-A1-93/19205
- WO-A1-2011/039425
- US-A1- 2010 144 544
- US-A1- 2012 064 511
- RACHEL LANGLAND ET AL: "Development of a multichannel TaqMan assay to simultaneously evaluate EGFR expression level and mutation status", CLINICAL CANCER RESEARCH, vol. 12, 1 October 2006 (2006-10-01), page A110, XP55171729,

## Description

### FIELD OF THE INVENTION

The present invention is related to the field of nucleic acid amplification by the polymerase chain reaction (PCR) assay. In particular, the invention pertains to use of fluorescent dyes for the identification of reagents that are used to perform PCR assays.

### BACKGROUND OF THE INVENTION

PCR is a powerful technique for amplifying DNA or RNA that can be used for a wide variety of purposes. The myriad of applications of PCR include its usage for the diagnosis of viral or bacterial genes and the identification of genetic mutations. Currently, PCR assays can be performed in real-time, homogenous formats, e.g. the TaqMan® assay, and using instruments that can test up to four nucleic acid sequences simultaneously. In a typical TaqMan® assay, target nucleic acids are detected by use of quenched fluorescent probes that are cleaved during PCR amplification, resulting in an increase in the fluorescence signals. However, in order to control for variability that may occur during PCR and also for optimization of the sensitivity for a given PCR assay, different enzymes, metal cofactors and concentrations of the constituents required for performing PCR are often used. In practice, many of these constituents are pre-mixed into a single solution, called a mastermix, on a per-assay basis. Because of this, assays with the same fluorophores but with different constituents may be performed side-by side (e.g. in adjacent wells on a multiwell plate) and without prior identification, the reactions would be indistinguishable, meaning that a positive fluorescence signal may be misinterpreted as a positive result for the wrong target nucleic acid.

Presently, there is an absence of methodologies to combat the difficulties in assuring that a correct mastermix is used in a PCR assay for a given target nucleic acid. This problem is particularly critical in a clinical laboratory setting to ensure that no user error has occurred. Although the prevalence of these errors should be low, their occurrence may result in the generation of false positive or false negative results which are extremely important to attenuate. WO 2011/039425 discloses a method of colour coding reaction mixtures by adding dyes so as to allow their unique identification.

### SUMMARY OF THE INVENTION

The present invention addresses the need to create certainty in the proper preparation of reagents and mastermix solutions that are used in PCR assays and lead to a higher confidence in the data generated from such PCR assays. In one aspect the disclosure provides for a method for preparing a plurality of reaction mixtures for performing polymerase chain reaction (PCR) amplification of a plurality of target nucleic acids, comprising providing a first mastermix solution comprising at least one substance required for performing PCR amplification of a first target nucleic acid, and further comprising a first fluorescent dye and a second fluorescent dye present at a predetermined concentration ratio that is unique for said first mastermix solution; providing a second mastermix solution comprising at least one substance required for performing PCR amplification of a second target nucleic acid, and further comprising said first fluorescent dye and said second fluorescent dye present at a predetermined concentration ratio different from that in said first mastermix solution and that is unique for said second mastermix solution; adding said first mastermix solution to a first reaction mixture to perform PCR amplification of said first target nucleic acid; and adding said second mastermix solution to a second reaction mixture to perform PCR amplification of said second target nucleic acid.

In another aspect, the disclosure provides for a method for encoding the identity of a mastermix solution used for PCR amplification of a target nucleic acid, comprising mixing together in the mastermix solution a first fluorescent dye and a second fluorescent dye present at a predetermined concentration ratio that produces a fluorescence ratio of the first fluorescent dye over the second fluorescent dye that is distinguishable from fluorescence ratios produced from other mastermix solutions; detecting the fluorescence from said first fluorescent dye and from said second fluorescent dye; and determining said fluorescence ratio, thereby identifying said mastermix solution.

In yet another aspect, the disclosure provides for a kit for performing PCR amplification of a plurality of target nucleic acids, comprising a plurality of mastermix solutions wherein each one mastermix solution from the plurality of mastermix solutions comprises at least one substance required for performing PCR amplification of a specific target nucleic acid, and further comprises a first fluorescence dye and a second fluorescence dye; and wherein for said each one mastermix solution, said first fluorescence dye and said second fluorescence dye are present at a concentration ratio that is different and produces a fluorescence ratio that is distinguishable from every other mastermix solution from the plurality of mastermix solutions.

### BRIEF DESCRIPTION OF THE DRAWINGS

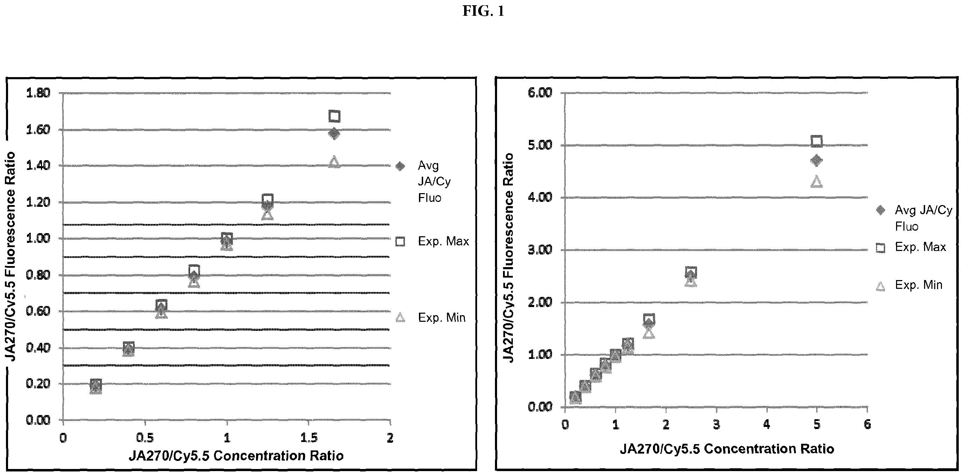

**FIG. 1** represents the graphs of the experiment conducted in Example 1 which shows the correlation between the concentration ratio and the fluorescence ratio for JA270/Cy5.5. The left graph represents a close-up view of the right graph at concentration ratios between 0 and 1.66.

### DETAILED DESCRIPTION OF THE INVENTION

### DEFINITIONS

The term "sample" as used herein includes a specimen or culture (e.g., microbiological cultures) that includes nucleic acids. The term "sample" is also meant to include both biological and environmental samples. A sample may include a specimen of synthetic origin. Biological samples include whole blood, serum, plasma, umbilical cord blood, chorionic villi, amniotic fluid, cerebrospinal fluid, spinal fluid, lavage fluid (e.g., bronchioalveolar, gastric, peritoneal, ductal, ear, arthroscopic), biopsy sample, urine, feces, sputum, saliva, nasal mucous, prostate fluid, semen, lymphatic fluid, bile, tears, sweat, breast milk, breast fluid, embryonic cells and fetal cells. In a preferred embodiment, the biological sample is blood, and more preferably plasma. As used herein, the term "blood" encompasses whole blood or any fractions of blood, such as serum and plasma as conventionally defined. Blood plasma refers to the fraction of whole blood resulting from centrifugation of blood treated with anticoagulants. Blood serum refers to the watery portion of fluid remaining after a blood sample has coagulated. Environmental samples include environmental material such as surface matter, soil, water and industrial samples, as well as samples obtained from food and dairy processing instruments, apparatus, equipment, utensils, disposable and non-disposable items.
The terms "target" or "target nucleic acid" as used herein are intended to mean any molecule whose presence is to be detected or measured or whose function, interactions or properties are to be studied. Therefore, a target includes essentially any molecule for which a detectable probe (e.g., oligonucleotide probe) or assay exists, or can be produced by one skilled in the art. For example, a target may be a biomolecule, such as a nucleic acid molecule, a polypeptide, a lipid, or a carbohydrate, that is capable of binding with or otherwise coming in contact with a detectable probe (e.g., an antibody), wherein the detectable probe also comprises nucleic acids capable of being detected. As used herein, "detectable probe" refers to any molecule or agent capable of hybridizing or annealing to a target biomolecule of interest and allows for the specific detection of the target biomolecule as described herein. In one aspect of the disclosure, the target is a nucleic acid, and the detectable probe is an oligonucleotide. The terms "nucleic acid" and "nucleic acid molecule" may be used interchangeably throughout the disclosure. The terms refer to oligonucleotides, oligos, polynucleotides, deoxyribonucleotide (DNA), genomic DNA, mitochondrial DNA (mtDNA), complementary DNA (cDNA), bacterial DNA, viral DNA, viral RNA, RNA, message RNA (mRNA), transfer RNA (tRNA), ribosomal RNA (rRNA), siRNA, catalytic RNA, clones, plasmids, M13, PI, cosmid, bacteria artificial chromosome (BAC), yeast artificial chromosome (YAC), amplified nucleic acid, amplicon, PCR product and other types of amplified nucleic acid, RNA/DNA hybrids and polyamide nucleic acids (PNAs), all of which can be in either single- or double-stranded form, and unless otherwise limited, would encompass known analogs of natural nucleotides that can function in a similar manner as naturally occurring nucleotides and combinations and/or mixtures thereof. Thus, the term "nucleotides" refers to both naturally-occurring and modified/nonnaturally-occurring nucleotides, including nucleoside tri, di, and monophosphates as well as monophosphate monomers present within polynucleic acid or oligonucleotide. A nucleotide may also be a ribo; 2'-deoxy; 2',3'-deoxy as well as a vast array of other nucleotide mimics that are well-known in the art. Mimics include chain-terminating nucleotides, such as 3'-O-methyl, halogenated base or sugar substitutions; alternative sugar structures including nonsugar, alkyl ring structures; alternative bases including inosine; deaza-modified; chi, and psi, linker-modified; mass label-modified; phosphodiester modifications or replacements including phosphorothioate, methylphosphonate, boranophosphate, amide, ester, ether; and a basic or complete internucleotide replacements, including cleavage linkages such a photocleavable nitrophenyl moieties.

The presence or absence of a target can be measured quantitatively or qualitatively. Targets can come in a variety of different forms including, for example, simple or complex mixtures, or in substantially purified forms. For example, a target can be part of a sample that contains other components or can be the sole or major component of the sample. Therefore, a target can be a component of a whole cell or tissue, a cell or tissue extract, a fractionated lysate thereof or a substantially purified molecule. Also a target can have either a known or unknown sequence or structure.

The term "amplification reaction" refers to any in vitro means for multiplying the copies of a target sequence of nucleic acid.

"Amplifying" refers to a step of submitting a solution to conditions sufficient to allow for amplification. Components of an amplification reaction may include, but are not limited to, e.g., primers, a polynucleotide template, nucleic acid polymerase, nucleotides, dNTPs and the like. The term "amplifying" typically refers to an "exponential" increase in target nucleic acid. However, "amplifying" as used herein can also refer to linear increases in the numbers of a select target sequence of nucleic acid, but is different than a one-time, single primer extension step.

"Polymerase chain reaction" or "PCR" refers to a method whereby a specific segment or subsequence of a target double-stranded DNA, is amplified in a geometric progression. PCR is well known to those of skill in the art; see, e.g., U.S. Patent Nos. 4,683,195 and 4,683,202; and PCR Protocols: A Guide to Methods and Applications, Innis et al., eds, 1990.

"Oligonucleotide" as used herein refers to linear oligomers of natural or modified nucleosidic monomers linked by phosphodiester bonds or analogs thereof. Oligonucleotides include deoxyribonucleosides, ribonucleosides, anomeric forms thereof, peptide nucleic acids (PNAs), and the like, capable of specifically binding to a target nucleic acid. Usually monomers are linked by phosphodiester bonds or analogs thereof to form oligonucleotides ranging in size from a few monomeric units, e.g., 3-4, to several tens of monomeric units, e.g., 40-60. Whenever an oligonucleotide is represented by a sequence of letters, such as "ATGCCTG," it will be understood that the nucleotides are in 5'-3' order from left to right and that "A" denotes deoxyadenosine, "C" denotes deoxycytidine, "G" denotes deoxyguanosine, "T" denotes deoxythymidine, and "U" denotes the ribonucleoside, uridine, unless otherwise noted. Usually oligonucleotides comprise the four natural deoxynucleotides; however, they may also comprise ribonucleosides or non-natural nucleotide analogs. Where an enzyme has specific oligonucleotide or polynucleotide substrate requirements for activity, e.g., single stranded DNA, RNA/DNA duplex, or the like, then selection of appropriate composition for the oligonucleotide or polynucleotide substrates is well within the knowledge of one of ordinary skill.
As used herein "oligonucleotide primer", or simply "primer", refers to a polynucleotide sequence that hybridizes to a sequence on a target nucleic acid template and facilitates the detection of an oligonucleotide probe. In amplification embodiments of the disclosure, an oligonucleotide primer serves as a point of initiation of nucleic acid synthesis. In non-amplification embodiments, an oligonucleotide primer may be used to create a structure that is capable of being cleaved by a cleavage agent. Primers can be of a variety of lengths and are often less than 50 nucleotides in length, for example 12-25 nucleotides, in length. The length and sequences of primers for use in PCR can be designed based on principles known to those of skill in the art.
The term " oligonucleotide probe" as used herein refers to a polynucleotide sequence capable of hybridizing or annealing to a target nucleic acid of interest and allows for the specific detection of the target nucleic acid.
The term "reagent solution" is any solution containing at least one reagent needed or used for PCR purposes. Most typical ingredients are polymerase, nucleotide, primer, ions, magnesium, salts, pH buffering agents, deoxynucleotide triphosphates (dNTPs), probe, fluorescent dye (may be attached to probe), nucleic acid binding agent, a nucleic acid template. The reagent may also be other polymerase reaction additive, which has an influence on the polymerase reaction or its monitoring.

The term "mastermix" refers to a mixture of all or most of the ingredients or factors necessary for PCR to occur, and in some cases, all except for the template and primers which are sample and amplicon specific. Commercially available mastermixes are usually concentrated solutions. A mastermix may contain all the reagents common to multiple samples, but it may also be constructed for one sample only. Using mastermixes helps to reduce pipetting errors and variations between samples due to differences between pipetted volumes.

A "nucleic acid polymerase" refers to an enzyme that catalyzes the incorporation of nucleotides into a nucleic acid. Exemplary nucleic acid polymerases include DNA polymerases, RNA polymerases, terminal transferases, reverse transcriptases, telomerases and the like.

A "thermostable DNA polymerase" refers to a DNA polymerase that is stable (i.e., resists breakdown or denaturation) and retains sufficient catalytic activity when subjected to elevated temperatures for selected periods of time. For example, a thermostable DNA polymerase retains sufficient activity to effect subsequent primer extension reactions, when subjected to elevated temperatures for the time necessary to denature double-stranded nucleic acids. Heating conditions necessary for nucleic acid denaturation are well known in the art and are exemplified in U.S. Patent Nos. 4,683,202 and 4,683,195. As used herein, a thermostable polymerase is typically suitable for use in a temperature cycling reaction such as the polymerase chain reaction ("PCR"). The examples of thermostable nucleic acid polymerases include Thermus aquaticus Taq DNA polymerase, Thermus sp. Z05 polymerase, Thermus flavus polymerase, Thermotoga maritima polymerases, such as TMA-25 and TMA-30 polymerases, Tth DNA polymerase, and the like.

A "modified" polymerase refers to a polymerase in which at least one monomer differs from the reference sequence, such as a native or wild-type form of the polymerase or another modified form of the polymerase. Exemplary modifications include monomer insertions, deletions, and substitutions. Modified polymerases also include chimeric polymerases that have identifiable component sequences (e.g., structural or functional domains, etc.) derived from two or more parents. Also included within the definition of modified polymerases are those comprising chemical modifications of the reference sequence. The examples of modified polymerases include G46E E678G CS5 DNA polymerase, G46E L329A E678G CS5 DNA polymerase, G46E L329A D640G S671F CS5 DNA polymerase, G46E L329A D640G S671F E678G CS5 DNA polymerase, a G46E E678G CS6 DNA polymerase, Z05 DNA polymerase, ΔZ05 polymerase, ΔZ05-Gold polymerase, ΔZ05R polymerase, E615G Taq DNA polymerase, E678G TMA-25 polymerase, E678G TMA-30 polymerase, and the like.

The term "5' to 3' nuclease activity" or "5'-3' nuclease activity" refers to an activity of a nucleic acid polymerase, typically associated with the nucleic acid strand synthesis, whereby nucleotides are removed from the 5' end of nucleic acid strand, e.g., E. coli DNA polymerase I has this activity, whereas the Klenow fragment does not. Some enzymes that have 5' to 3' nuclease activity are 5' to 3' exonucleases. Examples of such 5' to 3' exonucleases include: Exonuclease from *B. subtilis,* Phosphodiesterase from spleen, Lambda exonuclease, Exonuclease II from yeast, Exonuclease V from yeast, and Exonuclease from *Neurospora crassa.*

The detection of a target nucleic acid utilizing the 5' to 3' nuclease activity can be performed by a "TaqMan®" or "5'-nuclease assay", as described in U.S. Patent Nos. 5,210,015; 5,487,972; and 5,804,375; and Holland et al., 1988, Proc. Natl. Acad. Sci. USA 88: 7276-7280. In the TaqMan® assay, labeled detection probes that hybridize within the amplified region are present during the amplification reaction. The probes are modified so as to prevent the probes from acting as primers for DNA synthesis. The amplification is performed using a DNA polymerase having 5' to 3' exonuclease activity. During each synthesis step of the amplification, any probe which hybridizes to the target nucleic acid downstream from the primer being extended is degraded by the 5' to 3' exonuclease activity of the DNA polymerase. Thus, the synthesis of a new target strand also results in the degradation of a probe, and the accumulation of degradation product provides a measure of the synthesis of target sequences.

Any method suitable for detecting degradation product can be used in a 5' nuclease assay. Often, the detection probe is labeled with two fluorescent dyes, one of which is capable of quenching the fluorescence of the other dye. The dyes are attached to the probe, typically with the reporter or detector dye attached to the 5' terminus and the quenching dye attached to an internal site, such that quenching occurs when the probe is in an unhybridized state and such that cleavage of the probe by the 5' to 3' exonuclease activity of the DNA polymerase occurs in between the two dyes. Amplification results in cleavage of the probe between the dyes with a concomitant elimination of quenching and an increase in the fluorescence observable from the initially quenched dye. The accumulation of degradation product is monitored by measuring the increase in reaction fluorescence. U.S. Patent Nos. 5,491,063 and 5,571,673 describe alternative methods for detecting the degradation of a probe which occurs concomitant with amplification.

Fluorescent dyes may include dyes that are negatively charged, such as dyes of the fluorescein family, or dyes that are neutral in charge, such as dyes of the rhodamine family, or dyes that are positively charged, such as dyes of the cyanine family. Dyes of the fluorescein family include, e.g., 6-carboxy-fluorescein (FAM), 2', 4, 4', 5', 7, 7'-hexachlorofluorescein (HEX), TET, JOE, NAN and ZOE. Dyes of the rhodamine family include, e.g., Texas Red, ROX, R110, R6G, and TAMRA or the rhodamine derivative JA270 (see, US Patent No. 6,184,379). FAM, HEX, TET, JOE, NAN, ZOE, ROX, R110, R6G, and TAMRA are commercially available from, e.g., Perkin-Elmer, Inc. (Wellesley, MA, USA), and Texas Red is commercially available from, e.g., Molecular Probes, Inc. (Eugene, OR). Dyes of the cyanine family include, e.g., Cy2, Cy3, Cy5, Cy 5.5 and Cy7, and are commercially available from, e.g., Amersham Biosciences Corp. (Piscataway, NJ, USA).

A 5' nuclease assay for the detection of a target nucleic acid can employ any polymerase that has a 5' to 3' exonuclease activity. Thus, in some embodiments, the polymerases with 5'-nuclease activity are thermostable and thermoactive nucleic acid polymerases. Such thermostable polymerases include, but are not limited to, native and recombinant forms of polymerases from a variety of species of the eubacterial genera *Thermus, Thermatoga,* and *Thermosipho,* as well as chimeric forms thereof For example, *Thermus* species polymerases that can be used in the methods of the invention include *Thermus aquaticus* (*Taq*) DNA polymerase, *Thermus thermophilus* (*Tth*) DNA polymerase, *Thermus* species Z05 (Z05) DNA polymerase, *Thermus* species sps17 (sps17), and *Thermus* species Z05 (e.g., described in U.S. Patent Nos. 5,405,774; 5,352,600; 5,079,352; 4,889,818; 5,466,591; 5,618,711; 5,674,738, and 5,795,762). *Thermatoga* polymerases that can be used in the methods of the invention include, for example, *Thermatoga maritima* DNA polymerase and *Thermatoga neapolitana* DNA polymerase, while an example of a *Thermosipho* polymerase that can be used is *Thermosipho africanus* DNA polymerase. The sequences of *Thermatoga maritima* and *Thermosipho africanus* DNA polymerases are published in International Patent Publication No. WO 92/06200. The sequence of *Thermatoga neapolitana* may be found in International Patent Publication No. WO 97/09451.

In the 5' nuclease assay, the amplification detection is typically concurrent with amplification (i.e., "real-time"). In some embodiments the amplification detection is quantitative, and the amplification detection is real-time. In some embodiments, the amplification detection is qualitative (e.g., end-point detection of the presence or absence of a target nucleic acid). In some embodiments, the amplification detection is subsequent to amplification. In some embodiments, the amplification detection is qualitative, and the amplification detection is subsequent to amplification.

The present invention presents an opportunity to internally encode each reaction mixture used in a PCR assay to create certainty in the proper reaction preparation as well as higher confidence in the resulting data, e.g., is more reliable. The methods of this invention utilize a fluorescence-based approach which would allow for the automation of pre-mixed mastermix identification by instruments that can detect fluorescent signals.

The invention is the utilization of two different unquenched fluorescent dyes that are present in different concentration ratios that, in turn, gives rise to unique fluorescence ratios of the two dyes. These dyes must be uniquely different from the fluorescent dyes used by PCR probes, as the probes emit low fluorescence even in the presence of a quencher molecule. Emphasis is placed on the use of a fluorescence ratio rather than raw fluorescence because the ratio will be unchanged even if errors such as under-pipetting or over-pipetting the pre-made mastermix occur. Such errors would create changes in the raw fluorescence values, but the fluorescence ratios would remain constant. Thus, a mastermix for performing PCR amplification of a specific target nucleic acid may be designated with a predetermined ratio of the first fluorescent dye and the second fluorescent dye, for example, said ratio is between zero and five or one unit of the first dye to four units of the second dye. The fluorescence is read by a PCR instrument which can detect fluorescent signals, e.g. the LightCycler® instrument (Roche Diagnostics), and yield fluorescence values in a ratio of approximately one to four, respective of the two dyes. This allows the PCR instrument to identify the target-specific assay for which the specific mastermix is used. A wide span of concentration ratios between the first dye and the second dye can be used, with care given such that a concentration ratio for any given mastermix will not be too similar to a concentration ratio for other mastermixes. By using the methods of this invention, a PCR instrument such as the LightCycler® 480 instrument (Roche Diagnostics, Indianapolis, IN) can determine and report the target-specific assay, providing automated, useful information to clinical laboratories for confirmation and quality control purposes.

Inclusive in the utilized concentration ratio between the first dye and the second dye would be zero amount of the first dye, zero amount of the second dye, and/or zero amounts of both first and second dyes. The LightCycler® instrument would recognize the zero quantity of a dye by raw fluorescence abundance being well below the limit of detection for the dye. The lowest concentration of dye used should still be significantly above the limit of detection in order to minimize background noise and ensure that even in under-pipetting situations, the mastermix would remain accurately identified by the LightCycler® instrument.

The following examples and figures are provided to aid the understanding of the present invention, the true scope of which is set forth in the appended claims.

### EXAMPLES

### Use of JA270 and Cy5.5

An experiment was performed to determine if different concentration ratios of two fluorescent dyes, JA270 and Cy5.5, can yield fluorescence ratios that would allow the PCR analysis and detection instrument (e.g. LightCycler® instrument from Roche Diagnostics,) or a fluorescence reader to distinguish and identify the different concentration ratios. Twelve different concentration ratios of JA270 and Cy5.5 were used with amounts ranging from 0 pmol to 20 pmol in 100 µl of water. The different concentration ratios that were tested are shown in Table 1.

**TABLE 1**

| | | | | | | | | | | | | |
|---|---|---|---|---|---|---|---|---|---|---|---|---|
| JA270 (pmol) | 0 | 20 | 20 | 20 | 20 | 20 | 20 | 16 | 12 | 8 | 4 | 0 |
| Cy5.5 (pmol) | 0 | 0 | 4 | 8 | 12 | 16 | 20 | 20 | 20 | 20 | 20 | 20 |

Three replicates were prepared in a 96-well microtiter plate to examine for precision and reproducibility. Fluorescence was measured in a BMG PolarStar instrument using excitation wavelength of 610 nm and emission wavelength of 650 nm for JA270 and excitation wavelength of 670 nm and emission wavelength of 710 nm for Cy5.5. The results of this experiment are shown on FIG. 1 with the right graph showing on the x-axis the JA270/Cy5.5 concentration ratios between 0 and 5 and the left graph showing a close-up view of the JA270/Cy5.5 concentration ratios between 0 and 1.6. To determine the maximum possible expected variability based on these data, the maximum JA270 fluorescence value from each set of replicates was compared to the minimum Cy5.5 fluorescence value for each corresponding set of replicates. This was also done comparing the minimum JA270 fluorescence value to the maximum Cy5.5 value. The data showed that there was no overlap between any maxima or minima which meant that each concentration ratio tested was discernable from the other concentration ratios as identified by the fluorescence ratio, thereby validating the use of this method for identifying different solutions such as PCR mastermixes.

## Claims

1. A method for preparing a plurality of reaction mixtures for performing polymerase chain reaction (PCR) amplification of a plurality of target nucleic acids, comprising:
providing a first mastermix solution comprising at least one substance required for performing PCR amplification of a first target nucleic acid, and further comprising a first fluorescent dye and a second fluorescent dye, wherein said first dye and said second dye are present at a predetermined concentration ratio that is unique for said first reagent solution;
providing a second mastemix solution comprising at least one substance required for performing PCR amplification of a second target nucleic acid, and further comprising said first fluorescent dye and said second fluorescent dye, wherein said first dye and said second dye are present at a predetermined concentration ratio different from the concentration ratio for said first mastermix solution and that is unique for said second mastermix solution;
adding said first mastermix solution to a first reaction mixture to perform PCR amplification of said first target nucleic acid; and
adding said second mastermix solution to a second reaction mixture to perform PCR amplification of said second target nucleic acid, wherein the predetermined molar concentration ratio is between 0 and 5, the first fluorescent dye is JA270 and the second fluorescent dye is Cy5.5.

2. The method of claim 1, wherein in either the first mastermix solution or the second mastermix solution, the predetermined molar concentration ratio is between 0,2 and 5.

3. A method for encoding the identity of a mastermix solution used for performing polymerase chain reaction (PCR) amplification of a target nucleic acid, comprising:
mixing together in the mastermix solution a first fluorescent dye and a second fluorescent dye present at a predetermined concentration ratio that produces a fluorescence ratio of the first fluorescent dye over the second fluorescent dye that is distinguishable from fluorescence ratios produced from other mastermix solutions;
detecting the fluorescence from said first fluorescent dye and from said second fluorescent dye; and
determining said fluorescence ratio, thereby identifying said mastermix solution, wherein the predetermined molar concentration ratio is between 0 and 5, the first fluorescent dye is JA270 and the second fluorescent dye is Cy5.5.

4. The method of claim 3, wherein the predetermined molar ratio is between 0,2 and 5.

5. A kit for performing polymerase chain reaction (PCR) amplification of a plurality of target nucleic acids, comprising a plurality of mastermix solutions wherein each one mastermix solution from the plurality of mastermix solutions comprises at least one substance required for performing PCR amplification of a specific target nucleic acid, and further comprises a first fluorescence dye and a second fluorescence dye; and wherein for said each one mastermix solution, said first fluorescence dye and said second fluorescence dye are present at a concentration ratio that is different and produces a fluorescence ratio that is distinguishable from every other mastermix solution from the plurality of mastermix solutions, wherein the molar concentration ratio is between 0,2 and 5, the first fluorescent dye is JA270 and the second fluorescent dye is Cy5.5.

## Patentansprüche

1. Verfahren zur Herstellung einer Vielzahl von Reaktionsgemischen zur Durchführung einer Polymerasekettenreaktion(PCR)-Amplifikation einer Vielzahl von Zielnukleinsäuren, umfassend:
Bereitstellen einer ersten Mastermix-Lösung, die zumindest eine Substanz umfasst, die für die Durchführung einer PCR-Amplifikation einer ersten Zielnukleinsäure erforderlich ist, und außerdem einen ersten fluoreszierenden Farbstoff und einen zweiten fluoreszierenden Farbstoff umfasst, wobei der erste Farbstoff und der zweite Farbstoff in einem vorbestimmten Konzentrationsverhältnis vorhanden sind, das für die erste Reagenslösung einzigartig ist;
Bereitstellen einer zweiten Mastermix-Lösung, die zumindest eine Substanz umfasst, die für die Durchführung einer PCR-Amplifikation einer zweiten Zielnukleinsäure erforderlich ist, und außerdem den ersten fluoreszierenden Farbstoff und den zweiten fluoreszierenden Farbstoff umfasst, wobei der erste Farbstoff und der zweite Farbstoff in einem vorbestimmten Konzentrationsverhältnis vorhanden sind, das sich vom Konzentrationsverhältnis für die erste Mastermix-Lösung unterscheidet und einzigartig für die zweite Mastermix-Lösung ist;
Zusetzen der ersten Mastermix-Lösung zu einem ersten Reaktionsgemisch, um eine PCR-Amplifikation der ersten Zielnukleinsäure durchzuführen; und
Zusetzen der zweiten Mastermix-Lösung zu einem zweiten Reaktionsgemisch, um eine PCR-Amplifikation der zweiten Zielnukleinsäure durchzuführen, wobei das vorbestimmte molare Konzentrationsverhältnis zwischen 0 und 5 beträgt, der erste fluoreszierende Farbstoff JA270 ist und der zweite fluoreszierende Farbstoff Cy5.5 ist.

2. Verfahren nach Anspruch 1, wobei entweder in der ersten Mastermix-Lösung oder in der zweiten Mastermix-Lösung das vorbestimmte molare Konzentrationsverhältnis zwischen 0,2 und 5 beträgt.

3. Verfahren zur Codierung der Identität einer Mastermix-Lösung, die zur Durchführung einer Polymerasekettenreaktion(PCR)-Amplifikation einer Zielnukleinsäure verwendet wird, umfassend:
gemeinsames Einmischen eines ersten fluoreszierenden Farbstoffs und eines zweiten fluoreszierenden Farbstoffs in die Mastermix-Lösung, die in einem vorbestimmten Konzentrationsverhältnis vorhanden sind, das ein Fluoreszenzverhältnis zwischen dem ersten fluoreszierenden Farbstoff und dem zweiten fluoreszierenden Farbstoff erzeugt, das von Fluoreszenzverhältnissen unterscheidbar ist, die von anderen Mastermix-Lösungen erzeugt werden;
Detektieren der Fluoreszenz des ersten fluoreszierenden Farbstoffs und des zweiten fluoreszierenden Farbstoffs; und
Bestimmen des Fluoreszenzverhältnisses, wodurch die Mastermix-Lösung identifiziert wird, wobei das vorbestimmte molare Konzentrationsverhältnis zwischen 0 und 5 beträgt, der erste fluoreszierende Farbstoff JA270 ist und der zweite fluoreszierende Farbstoff Cy5.5 ist.

4. Verfahren nach Anspruch 3, wobei das vorbestimmte Molverhältnis zwischen 0,2 und 5 beträgt.

5. Kit zur Durchführung einer Polymerasekettenreaktion(PCR)-Amplifikation einer Vielzahl von Zielnukleinsäuren, umfassend eine Vielzahl von Mastermix-Lösungen, wobei jede Mastermix-Lösung aus der Vielzahl von Mastermix-Lösungen zumindest eine Substanz umfasst, die für die Durchführung einer PCR-Amplifikation einer spezifischen Zielnukleinsäure erforderlich ist, und außerdem einen ersten fluoreszierenden Farbstoff und einen zweiten fluoreszierenden Farbstoff umfasst; und wobei in jeder Mastermix-Lösung der erste fluoreszierende Farbstoff und der zweite fluoreszierende Farbstoff in einem Konzentrationsverhältnis vorhanden sind, das unterschiedlich ist und ein Fluoreszenzverhältnis erzeugt, das von jeder anderen Mastermix-Lösung aus der Vielzahl von Mastermix-Lösungen unterscheidbar ist, wobei das molare Konzentrationsverhältnis zwischen 0,2 und 5 beträgt, der erste fluoreszierende Farbstoff JA270 ist und der zweite fluoreszierende Farbstoff Cy5.5 ist.

## Revendications

1. Procédé pour préparer une pluralité de mélanges réactionnels pour effectuer une amplification par réaction en chaîne par polymérase (PCR) d'une pluralité d'acides nucléiques cibles, comprenant :
l'utilisation d'une première solution de mélange-maître comprenant au moins une substance nécessaire pour effectuer une amplification par PCR d'un premier acide nucléique cible et comprenant en outre un premier colorant fluorescent et un second colorant fluorescent, ledit premier colorant et ledit second colorant étant présents selon un rapport des concentrations prédéterminé qui est unique pour ladite première solution de réactif ;
l'utilisation d'une seconde solution de mélange-maître comprenant au moins une substance nécessaire pour effectuer une amplification par PCR d'un second acide nucléique cible et comprenant en outre ledit premier colorant fluorescent et ledit second colorant fluorescent, ledit premier colorant et ledit second colorant étant présents selon un rapport des concentrations prédéterminé différent du rapport des concentrations pour ladite première solution de mélange-maître et qui est unique pour ladite seconde solution de mélange-maître ;
l'ajout de ladite première solution de mélange-maître à un premier mélange réactionnel pour effectuer une amplification par PCR dudit premier acide nucléique cible ; et
l'ajout de ladite seconde solution de mélange-maître à un second mélange réactionnel pour effectuer une amplification par PCR dudit second acide nucléique cible, le rapport des concentrations molaires prédéterminé étant compris entre 0 et 5, le premier colorant fluorescent étant JA270 et le second colorant fluorescent étant Cy5.5.

2. Procédé selon la revendication 1, dans lequel, dans la première solution de mélange-maître ou dans la seconde solution de mélange-maître, le rapport des concentrations molaires prédéterminé est compris entre 0,2 et 5.

3. Procédé pour coder l'identité d'une solution de mélange-maître utilisée pour effectuer une amplification par réaction en chaîne par polymérase (PCR) d'un acide nucléique cible, comprenant :
le mélange ensemble, dans la solution de mélange-maître, d'un premier colorant fluorescent et d'un second colorant fluorescent présents selon un rapport des concentrations prédéterminé qui produit un rapport des fluorescences du premier colorant fluorescent et du second colorant fluorescent qui se distingue des rapports des fluorescences produits à partir d'autres solutions de mélange-maître ;
la détection de la fluorescence dudit premier colorant fluorescent et dudit second colorant fluorescent ; et
la détermination dudit rapport des fluorescences, identifiant ainsi ladite solution de mélange-maître, le rapport des concentrations molaires prédéterminé étant compris entre 0 et 5, le premier colorant fluorescent étant JA270 et le second colorant fluorescent étant Cy5.5.

4. Procédé selon la revendication 3, dans lequel le rapport molaire prédéterminé est compris entre 0,2 et 5.

5. Kit pour effectuer une amplification par réaction en chaîne par polymérase (PCR) d'une pluralité d'acides nucléiques cibles, comprenant une pluralité de solutions de mélange-maître, chacune des solutions de mélange-maître de la pluralité de solutions de mélange-maître comprenant au moins une substance nécessaire pour effectuer une amplification par PCR d'un acide nucléique cible spécifique et comprenant en outre un premier colorant fluorescent et un second colorant fluorescent ; et pour chacune desdites solutions de mélange-maître, ledit premier colorant de fluorescence et ledit second colorant de fluorescence étant présents selon un rapport des concentrations qui est différent et produit un rapport des fluorescences qui se distingue de celui de chaque autre solution de mélange-maître de la pluralité de solutions de mélange-maître, le rapport des concentrations molaires prédéterminé étant compris entre 0,2 et 5, le premier colorant fluorescent étant JA270 et le second colorant fluorescent étant Cy5.5.
